# EUROPÄISCHE PATENTANMELDUNG

(11) **EP 0 972 496 A2**
(43) Veröffentlichungstag der Anmeldung: **19.01.2000**
(21) Anmeldenummer: 99112691.3
(22) Anmeldetag: 02.07.1999
(51) Int. Cl.: A61F 2/00, A61F 2/04

(54) **Implantathalter**

(30) Priorität: 15.07.1998 DE 19831699
(71) Anmelder: CareMed Medical Produkte Aktiengesellschaft, 01109 Dresden (DE)
(72) Erfinder: Gerlach, Roland, Dr., 34302 Guxhagen (DE); Hannappel, Josef, Dr., 50259 Pulheim (DE); Reuter, Jürgen, 36211 Alheim (DE); Rohrmann, Dorothea, Dr., 52379 Langerwehe (DE)
(74) Vertreter: Dallmeyer, Georg, Dipl.-Ing.

(57) **Zusammenfassung**

Der Implantathalter weist eine Abschirmwand (21) auf, die an einer Seite aus einem gewebefreundlichen Fasermaterial (25) zum Anwachsen körpereigener Zellen besteht und an der anderen Seite eine glatte Anlagefläche (23) für das Implantat (10) aufweist. Eine Halterung (26) hält das Implantat (10) an der Abschirmwand (21) fest. Der Implantathalter verhindert, daß das Körpergewebe unmittelbar an dem Implantat anwächst, was ein Auswechseln des Implantats (10) erschweren würde. Zum Auswechseln wird die Haut (16) des Patienten aufgeschnitten und die Halterung (26) geöffnet. Der Implantathalter verbleibt im Körper, während das Implantat (10) gewechselt wird.

## Beschreibung

Die Erfindung betrifft einen Implantathalter für Körperimplantate, wie beispielsweise eine künstliche Harnblase, Insulinpumpen, einen Herzschrittmacher oder eine Medikamentenkapsel.

Implantate, die in den menschlichen oder tierischen Körper implantiert werden, benötigen häufig einen Zugang, beispielsweise zum Zwecke der Auswechslung, der Erneuerung des Inhalts oder zu Wartungszwecken. Normalerweise haben Implantate ein Gehäuse aus körperverträglichem Material, an das die körpereigenen Zellen im Laufe der Zeit anwachsen. Dadurch verbindet sich das Körpergewebe fest mit der Implantatkapsel. Wenn diese Implantatkapsel herausgenommen werden soll, muß zunächst das Körpergewebe abgetrennt werden. Ferner benötigen manche Implantate eine Halterung, damit sie den vorgesehenen Platz im Körper beibehalten. Eine solche Halterung ist in DE 27 60 437 C2 für eine prothetische Blase beschrieben. Die Halterung weist Bandagen auf, die am Bauchfell oder an der Muskulatur befestigt werden und die die prothetische Blase festhalten.

Der Erfindung liegt die Aufgabe zugrunde, einen Implantathalter zu schaffen, der es ermöglicht, Implantate im Bedarfsfall auf einfache Weise ohne wesentliche Belastung des Körpers auszuwechseln.

Die Lösung dieser Aufgabe erfolgt erfindungsgemäß mit den im Patentanspruch 1 angegebenen Merkmalen.

Der erfindungsgemäße Implantathalter weist eine Abschirmwand auf, an die das Körpergewebe von der einen Seite her anwächst, während die andere Seite eine Anlagefläche für das Implantat aufweist. Diese Anlagefläche ist vorzugsweise glatt bzw. gewebeabstoßend. Auf diese Weise wird in dem Körper subkutan eine Art Tasche oder Nest gebildet, um das Implantat aufzunehmen, ohne daß dieses vollständig umwachsen wird. An der Abschirmwand ist ferner eine Halterung vorgesehen, die geeignet ist, das Implantat an der Anlagefläche festzuhalten. Da Implantate meist unmittelbar unter der Haut positioniert werden, ist das Implantat durch einen einfachen Hautschnitt zugänglich und entnehmbar, ohne daß es an dem Körper haftet und zurückgehalten wird. Das Implantat ist in örtlicher Betäubung des Patienten zugänglich. Dieser benötigt also keine Vollnarkose, wie sie in der Regel dann erforderlich ist, wenn das Implantat von dem Körpergewebe abgetrennt werden muß.

Die Abschirmwand weist an der dem Implantat zugewandten Seite eine Anlagefläche und an der Außenseite eine Schicht aus gewebefreundlichem Fasermaterial, vorzugsweise einem Veloursmaterial, auf. Als Material für die glatte Anlagefläche eignet sich eine glatte Silikonfolie. Silikone sind gewebeverträglich, jedoch wird bei einer glatten Folie das Anwachsen erschwert.

Im folgenden wird unter Bezugnahme auf die Zeichnungen ein Ausführungsbeispiel der Erfindung näher erläutert.

Es zeigen:
- Fig. 1: eine Seitenansicht einer künstlichen Harnblase, die in den Körper implantiert ist,
- Fig. 2: eine Frontansicht der künstlichen Harnblase, die in den Implantathalter eingesetzt ist, und
- Fig. 3: einen Querschnitt durch das Material der Abschirmwand des Implantathalters.

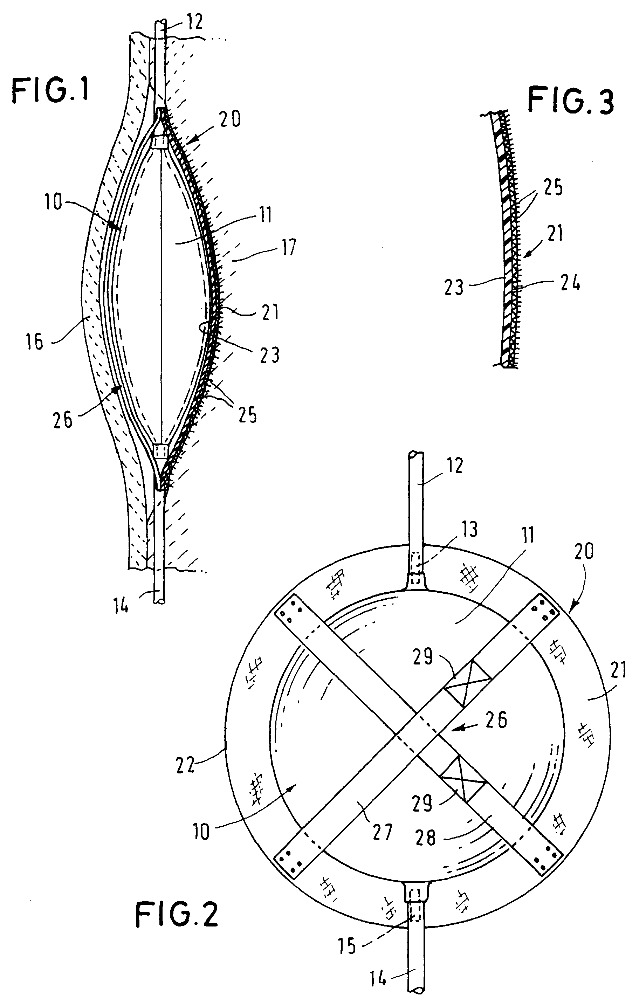

In Fig. 1 ist ein Implantat 10 dargestellt, bei dem es sich um eine künstliche Harnblase handelt. Das Implantat 10 besteht aus einem Behälter 11 aus elastischem Kunststoff. Dieser Behälter, der in Frontansicht (Fig. 2) rund ist, kann axial zusammengedrückt werden. Bei Beendigung der axialen Drückkraft bildet sich jedoch die ursprüngliche Behälterform wieder aus, in der der Behälter aufgeweitet ist.

In den Behälter 11 mündet ein von einer Niere kommender Katheter 12, der ein Rückschlagventil 13 enthält. Aus dem unteren Ende des Behälters 11 führt ein Harnröhrenkatheter 14 heraus, der ein Rückschlagventil 15 enthält. Der Behälter 11 wird zwischen der Haut 16 und dem Muskelgewebe 17 des Patienten implantiert. Dieser kann durch Druck von außen den Behälter 11 zusammendrücken, um darin befindliche Flüssigkeit in den Harnröhrenkatheter 14 hineinzudrücken. Anschließend ist der Behälter 11 bestrebt, sich wieder auszudehnen. Dadurch wirkt er eine Saugwirkung aus, um Harn aus der Niere abzusaugen.

Das Implantat 10 ist auswechselbar in den Implantathalter 20 eingesetzt. Der Implantathalter 20 weist eine Abschirmwand 21 auf, die das Implantat 10 von dem Muskelgewebe 17 trennt. Die Abschirmwand 21 ist so groß, daß sie die horizontale Projektion des Implantats 10 vollständig bedeckt und noch überragt, wie aus Fig. 2 hervorgeht, so daß die Abschirmwand mit einem überstehenden Rand 22 über die Kontur des Implantats 10 vorsteht.

Die Abschirmwand 21 weist an der dem Implantat 10 zugewandten Innenseite eine Folienschicht 23 aus glatter Silikonfolie auf. Diese Folienschicht hat eine Stärke von 1 - 1,5 mm. An der Außenseite der Folienschicht 23 befindet sich eine Veloursschicht 24 aus gewebefreundlichem Fasermaterial, vorzugsweise aus Dakron-Velours. Die Härchen dieser Veloursschicht 24 stehen nach außen ab und bilden das Fasermaterial 25 zum Anwachsen des Muskelgewebes 17.

An dem Rand 22 der Abschirmwand 21 ist eine Halterung 26 befestigt, die das Implantat 10 an der Innenseite der Abschirmwand 21 festhält. Diese Halterung 26 besteht hier aus zwei kreuzförmig angeordneten Bandagen 27,28, die jeweils mit einem Schloß 29 versehen sind. Die Bandagen 27,28 bestehen aus relativ schmalen Bändern aus einem Material, an dem Zellen nicht gut anwachsen können. Auch das Material des Implantats 10 ist so gewählt, daß es sich mit dem Körpergewebe möglichst nicht verbindet.

Während die eine Seite des Implantats durch die Abschirmwand vollständig bedeckt ist, liegt die andere Seite des Implantats frei, wobei sie zu einem geringen Teil (weniger als 10 %) lediglich durch die Halterung 26 bedeckt ist. Alternativ besteht die Möglichkeit, die Halterung an der Abschirmwand 21 vorzusehen, so daß dann die der Abschirmwand abgewandte Fläche des Implantats völlig freiliegt.

Sollte es erforderlich sein, das Implantat 10 auszuwechseln, so wird durch einen oder mehrere Schnitte lediglich die Haut 16 geöffnet. Dann ist nach dem Öffnen der Schlösser 29 das Implantat 10 zum Herausnehmen zugänglich. Der Implantathalter 20 verbleibt im Körper, während das Implantat 10 herausgenommen und ggf. ersetzt wird. Danach muß lediglich die Haut 16 wieder geschlossen und vernäht werden.

Falls das Implantat 10, wie bei dem vorliegenden Ausführungsbeispiel, eine künstliche Harnblase ist, die mit Kathetern 12,14 verbunden ist, sollte die Katheterverbindung lösbar sein, damit die Katheter implantiert bleiben können, während die Harnblase ausgewechselt wird.

## Patentansprüche

1. Implantathalter für Körperimplantate, mit einer anwachsenden Abschirmwand (21), die an einer Seite gewebefreundliches Fasermaterial (25) zum Anwachsen körpereigener Zellen und an der gegenüberliegenden Seite eine Anlagefläche für das Implantat (10) aufweist, und daß eine Halterung (26) vorgesehen ist, die geeignet ist, ein Implantat (10) an der Anlagefläche festzuhalten.

2. Implantathalter nach Anspruch 1, dadurch gekennzeichnet, daß die Abschirmwand (21) die horizontale Projektion des Implantats vollständig bedeckt.

3. Implantathalter nach Anspruch 1 oder 2, dadurch gekennzeichnet, daß die Halterung (26) aus mindestens einer an der Abschirmwand (21) befestigten Bandage (27,28) besteht.

4. Implantathalter nach einem der Ansprüche 1-3, dadurch gekennzeichnet, daß die Abschirmwand (21) aus einer Folie (23) und einer Veloursschicht (24) besteht.
